# EUROPEAN PATENT APPLICATION

(11) **EP 4 162 964 A1**
(43) Date of publication of application: **12.04.2023**
(21) Application number: 21818045.3
(22) Date of filing: 14.04.2021
(51) Int. Cl.: A61M 5/30

(54) **NEEDLELESS SYRINGE**

(30) Priority: 05.06.2020 JP 2020098730
(71) Applicant: Daicel Corporation, Osaka 530-0011 (JP)
(72) Inventor: SUZUKI, Takamasa, Tokyo 108-8230 (JP)
(74) Representative: Grünecker Patent- und Rechtsanwälte PartG mbB
(86) International application number: PCT/JP2021/015393
(87) International publication number: WO 2021/246061

(57) **Abstract**

A needleless injector configured to eject an injection substance to a target region without using an injection needle, and includes an injector main body, a housing part including an accommodating space in which the injection substance is accommodated, the housing part defining a flow path to enable the injection substance to be ejected to the target region through an ejection port, and an attachment part configured to attach the housing part to the injector main body. The attachment part attaches the housing part to the injector main body, with a distal end side outer surface of the housing part corresponding to an inner surface of the housing part on a distal end side, where a communication portion where the flow path is in communication with the accommodating space is located, pressed in a direction opposite to a movement direction of a pressurizing unit that pressurizes the injection substance toward the ejection port.

## Description

### Technical Field

The present invention relates to a needleless injector that ejects an injection substance to a target region without using an injection needle.

### Background Art

An example of a device that ejects an injection substance such as a liquid chemical to a target region such as an organism includes a needleless injector using no injection needle has been attracting attention in terms of usability and sanitation, and thus has been actively developed recently. In general, a needleless injector has been brought into practical application and has a configuration in which liquid medicine pressurized by a driving source such as compressed gas or a spring is injected toward a target region, and the liquid medicine is injected into the inside of the target region through use of the kinetic energy of the liquid medicine.

In the needleless injector, relatively large ejection energy needs to be imparted to the injection substance, to inject the injection substance. Thus, a relatively large load is applied to a housing part that contains the injection substance before the energy is imparted, meaning that sufficient rigidity of the housing part needs to be secured. For example, the technique illustrated in Patent Document 1 uses a configuration in which a cylindrical sleeve is disposed that supports a side surface of a glass needleless injector capsule containing contents.

### Citation List

### Patent Document

Patent Document 1: JP H10-509613 T

### Summary of Invention

### Technical Problem

In a case where the injector is to function as a needleless injector with the injection substance pressurized for injection, the force for the pressurization needs to be relatively large. However, for the needleless injector to be used safely, the needleless injector needs to be favorably prevented from being damaged or deformed under the pressurization. In a known technique, the side surface of the glass capsule containing the content to be pressurized is reinforced with a sleeve. However, it has been found that with such a reinforcement configuration, it is difficult to sufficiently prevent damage or the like to the capsule.

In view of the problem described above, an object of the disclosure of the present application is to provide a reinforcement technique with which damage or the like to a needleless injector may be favorably prevented, when pressurizing an injection substance in the needleless injector.

### Solution to Problem

To solve the problem described above, a needleless injector of the disclosure of the present application employs a configuration in which a housing part that contains an injection substance to be pressurized for injection is attached to an injector main body, while being pressurized in a direction opposite to the direction of the pressurization of the injection substance. With such a housing part attachment configuration, deformation of the housing part at the time of the pressurization of the injection substance can be favorably suppressed, whereby damage or the like to the housing part can be prevented.

Specifically, the disclosure of the present application discloses a needleless injector that is configured to eject an injection substance to a target region without using an injection needle, the needleless injector including an injector main body, a housing part including an accommodating space in which the injection substance is accommodated, the housing part defining a flow path to enable the injection substance to be ejected to the target region through an ejection port, an attachment part configured to attach the housing part to the injector main body, a driving part that is provided in the injector main body and imparts ejection energy for ejecting the injection substance, and a pressurizing unit that is disposed to be movable in a direction toward the ejection port in a predetermined region that is provided to be located inside both the injector main body and the housing part, and pressurizes the injection substance accommodated in the accommodating space, upon being imparted with the ejection energy. The attachment part attaches the housing part to the injector main body, with a distal end side outer surface of the housing part corresponding to an inner surface of the housing part on a distal end side where a communication portion where the flow path is in communication with the accommodating space is located, pressed in a direction opposite to the movement direction of the pressurizing unit toward the ejection port.

In the needleless injector described above, the driving part imparts the ejection energy, and thus the injection substance is ejected to the target region. In the present application, "ejection" is achieved by pressurizing the injection substance in the housing part with the pressurizing unit using the ejection energy imparted by the driving part, to enable the injection substance to flow through the flow path in the housing part. In the needleless injector, the distal end side is a side relatively closer to the ejection port, and the base end side is a side opposite to the distal end side in the longitudinal direction of the needleless injector.

Further, as the injection substance ejected from the needleless injector, predetermined substances including a component expected to have effects in the target region or a component expected to exert a predetermined function in the target region can be exemplified. Thus, the injection substance may have any physical form, as long as the ejection by the ejection energy as described above can at least be performed. For example, the injection substance may be dissolved in a liquid, or may be simply mixed with a liquid without dissolving therein. As one example, the predetermined substance to be sent includes a vaccine for intensifying an antibody, a protein for cosmetic enhancement, a cultured cell for hair regeneration, and the like, and is included in a liquid medium in an ejectable manner. The injection substance is formed in this way. Note that the medium is preferably a medium that does not hinder the above-mentioned effect and function of the predetermined substance in a state of being injected into the target region. As another method, the medium may be a medium that exerts the above-mentioned effect and function by acting together with the predetermined substance in the state of being injected into the target region.

The injection substance ejected needs to penetrate the surface of the target region such that the injection substance is ejected from the needleless injector to the target region to be delivered into the inside thereof. Thus, at an initial stage of ejection, the injection substance needs to be ejected to the target region at a relatively high speed. In view of this point, as an example, the driving part preferably imparts the ejection energy using a combustion product discharged by combustion of an ignition charge. Note that, as the ignition charge, there may be employed any one of an explosive containing zirconium and potassium perchlorate, an explosive containing titanium hydride and potassium perchlorate, an explosive containing titanium and potassium perchlorate, an explosive containing aluminum and potassium perchlorate, an explosive containing aluminum and bismuth oxide, an explosive containing aluminum and molybdenum oxide, an explosive containing aluminum and copper oxide, an explosive containing aluminum and iron oxide, or an explosive composed of a combination of a plurality of the above explosives. As characteristics of the above-mentioned ignition charge, the combustion product is gas at a high temperature but does not include a gas component at a room temperature, hence the combustion product is condensed immediately after the ignition. As a result, the driving part can impart the ejection energy in an extremely short period of time. In addition, the driving part may utilize electrical energy of a piezoelectric element or the like or mechanical energy of a spring or the like as the ejection energy instead of the ejection energy caused by the combustion of the ignition charge, and may generate the ejection energy by appropriately combining these forms of energy.

In the needleless injector described above, the accommodating space in the housing part accommodates the injection substance. The pressurizing unit moves in a direction toward the ejection port to pressurize the injection substance. The housing part is attached to the injector main body via the attachment part. In a state where attachment is established with the attachment part, the distal end side outer surface of the housing part is in a state of being pressed in a direction opposite to the movement direction of the pressurizing unit. The applicant of the present application has newly found that when a load is applied due to the movement of the pressurizing unit, the housing part first deforms in the movement direction, and from the point of the deformation, damage or the like to the entire housing part occurs. In the specification of the present application, this deformation will be referred to as "initial deformation". With the attachment configuration described above employed, the housing part is supported along the movement direction of the pressurizing unit against the load applied to the injection substance when the pressurizing unit moves toward the ejection port in the predetermined region upon being imparted with ejection energy. Thus, the initial deformation that triggers the damage or the like of or the like to the entire housing part can be favorably suppressed.

The distal end side outer surface of the housing part is defined as an outer surface of the housing part corresponding to the inner surface of the housing part on the distal end side where a communication portion where the flow path is in communication with the accommodating space is located, that is, an outer surface facing a wall of the housing part including the inner surface of the housing part. The shape of the distal end side outer surface is not limited to a specific shape, and does not necessarily need to be parallel to the inner surface of the housing part.

The needleless injector employs this attachment configuration in which the housing part is attached using the attachment part, whereby the initial deformation of the housing part at the time of pressurization can be favorably suppressed. Thus, safe use of the needleless injector can be sufficiently secured. It suffices if the reinforcement of the housing part by the attachment part includes the reinforcement on the distal end side outer surface. Thus, the side surface of the housing part does not necessarily need to be reinforced. Still, this does not exclude the reinforcement of the side surface of the housing part. For the sake of reinforcement on the distal end side outer surface by the attachment part, the tensile strength of the attachment part is preferably not lower than the tensile strength of the housing part. With this configuration, the initial deformation described above can be more reliably suppressed.

Now, a description will be given regarding more specific aspects of the needleless injector described above. In a first aspect, the housing part may include: a base end portion radially expanded in a radial direction and including a base end surface; a distal end portion including the distal end side outer surface; and a body portion that connects the base end portion and the distal end portion to each other. In such a case, the needleless injector may be configured in such a manner that the attachment part is fastened to the injector main body with the base end portion sandwiched between the attachment part and the injector main body, to attach the housing part to the injector main body with the base end surface of the housing part on a side opposite to a distal end surface of the housing part where the ejection port is provided, brought into contact with a predetermined end surface that is part of a surface of the injector main body intersecting the predetermined region, and with the distal end side outer surface of the housing part pressed. The predetermined end surface in the injector main body is part of the surface of the injector main body that crosses the predetermined region in which the pressurizing unit moves. Thus, of a surface on the injector main body side and a surface on the housing part to be in contact with each other for the predetermined region to be located inside both the injector main body and the housing part, the surface on the injector main body side is the predetermined end surface. In a second aspect, the housing part may include: a base end portion where the attachment part is fastened to the housing part, the base end portion including a base end surface; a distal end portion including the distal end side outer surface; and a body portion that connects the base end portion and the distal end portion to each other. In such a case the needleless injector may be configured in such a manner that the attachment part is fastened to the housing part to make the attachment part be in contact with and press the distal end side outer surface of the distal end portion, and the housing part is attached to the injector main body by attaching the attachment part to which the housing part is fastened to the injector main body.

With these aspects, the distal end side outer surface of the housing part is reliably pressed, whereby the initial deformation described above can be favorably suppressed. Any aspect can be employed for fastening between the injector main body and the attachment part, and between the housing part and the attachment part. For example, mating, snap fastening, fastening using bolts, or the like can be used. The shape of the housing part is not limited to the shapes related to the two aspects described above. The housing part may have a shape other than the shapes described above, as long as a configuration enabling the attachment to the injector main body by the attachment part can be achieved.

In the needleless injector described above, the distal end portion may include: a protruding portion that includes part of the flow path on a side of the ejection port; and a tapered portion that connects the protruding portion and the body portion to each other and includes the distal end side outer surface inclined with respect to a center axis along a longitudinal direction of the injector main body. In such a case, the attachment part may have an opening formed, through which the protruding portion passes, to open the ejection port, and include a contact portion that comes into contact with a region having a predetermined width from a connection portion where the tapered portion and the body portion are connected to each other in the distal end side outer surface. The predetermined width may be a width covering the distal end side outer surface entirely, or may be a width covering the distal end side outer surface partially. With the distal end portion provided with the tapered portion, a distance from the base end portion to part of the distal end portion (the region of the predetermined width from the connection portion) required for pressing the distal end side outer surface can be shortened thereby reducing the size and weight of the attachment part.

In the needleless injector described above, a ratio of an area of the opening to a cross-sectional area of the body portion may be from 0.10 to 0.74. With such an aspect, damage or the like to the housing part at the time of pressurization can be favorably avoided. Thus, the aspect contributes to the safe use of the needleless injector.

### Advantageous Effects of Invention

According to the disclosure of the present application, damage or the like to the needleless injector can be favorably prevented, when the injection substance is pressurized in the needleless injector.

### Brief Description of Drawings

FIG. 1 is a diagram illustrating a schematic configuration of a needleless injector.
FIG. 2 is a diagram illustrating a schematic configuration of an actuator incorporated in the needleless injector.
FIG. 3 is a diagram illustrating a schematic configuration of an attachment incorporated in the needleless injector.
FIG. 4 is a diagram illustrating a first mode of attachment of a container relative to an injector main body in the needleless injector.
FIG. 5 is a diagram illustrating a dimensional relationship dimension between the container and a container holder in the needleless injector.
FIG. 6 is a diagram illustrating a variation of the container holder related to the first mode.
FIG. 7 is a diagram illustrating a second mode of attachment of the container to the injector main body in the needleless injector.
FIG. 8 is a diagram illustrating a variation of the container holder related to the second mode.

### Description of Embodiments

With reference to the drawings, a needleless injector 1 (hereinafter, simply referred to as "injector 1") without an injection needle is described as an example of an administration device of the disclosure of the present application. Note that each of the configurations, combinations thereof, and the like in each embodiment described in the present application is an example, and additions, omissions, substitutions, and other changes of the configuration may be made as appropriate without departing from the spirit of the present invention. The present disclosure is not limited by the embodiment, but only by the claims.

### First Embodiment

The injector 1 is a needleless injector that injects an ejection solution, which corresponds to an injection substance of the present application, into a target region, using the combustion energy of an explosive. In other words, the injector 1 is a device that injects the ejection solution into the target region without using an injection needle. Note that, in the present embodiment, as terms indicating a relative positional relationship in a longitudinal direction of the injector 1, "distal end side" and "base end side" are used. The "distal end side" indicates a side closer to the distal end of the injector 1 described below, that is, a position closer to an ejection port 77, and the "base end side" indicates a side in an opposite direction to the "distal end side" in a longitudinal direction of the injector 1, that is, a direction to an igniter 22 side of an actuator 20.

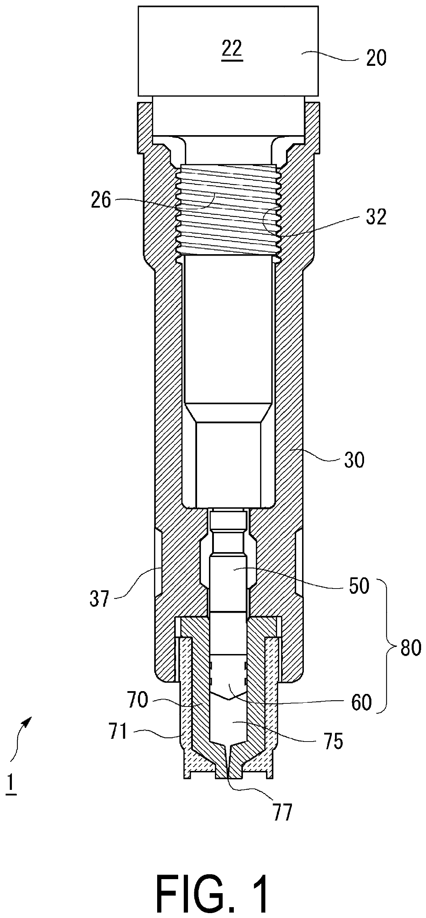

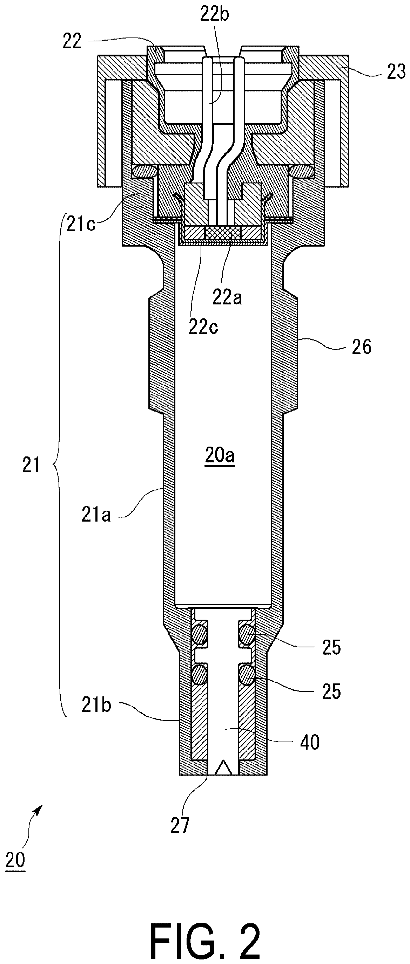

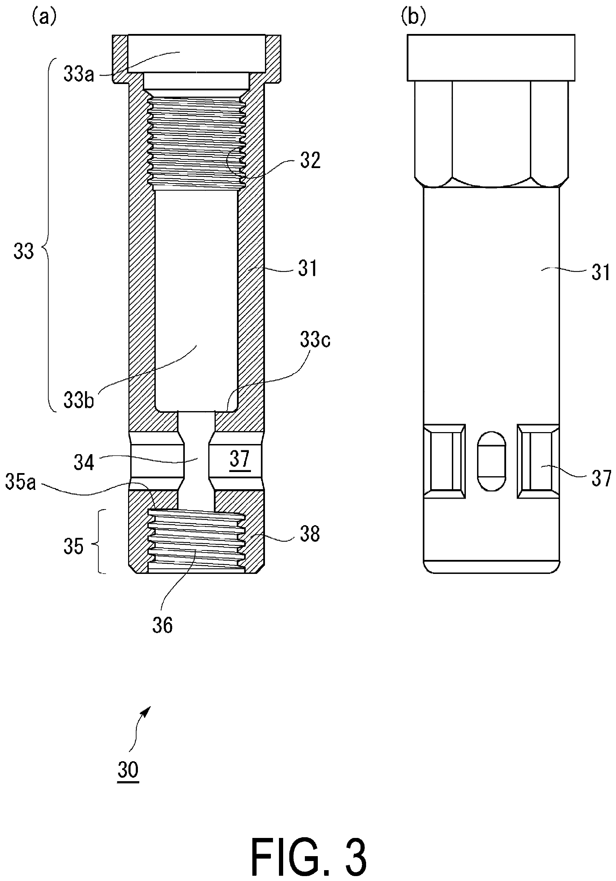

Here, FIG. 1 is a cross-sectional view of the injector 1 illustrating a schematic configuration of the injector 1. The injector 1 includes the actuator 20, an attachment 30, a container 70, a plunger 80, and the like. FIG. 2 is a cross-sectional view of the actuator 20 attached to the injector 1 illustrating a schematic configuration thereof. FIG. 3 is a diagram illustrating a schematic configuration of the attachment 30. An accommodating space 75 formed in the container 70 between the plunger 80 and the container 70 included in the injector 1 is filled with ejection solution during a preparation stage before the actuation of the injector 1. Note that, in the following description in the present application, the ejection solution ejected to the target region by the injector 1 is formed of a liquid medium including a predetermined substance, which exerts an expected effect or function in the target region. In the ejection solution, the predetermined substance may be in a state of being dissolved in liquid being a medium, or may be in a state of being simply mixed instead of being dissolved.

For example, examples of the predetermined substance included in the ejection solution include an organism-derived substance and a substance with a desired bioactivity, which can be ejected to the target region being an organism. For example, examples of the organism-derived substance include DNA, RNA, a nucleic acid, an antibody, and a cell. Examples of the substance with a desired bioactivity include various substances exerting pharmacological or therapeutic effects, which are exemplified by medicines composed of low molecular compounds, proteins, peptides, or the like, a vaccine, an inorganic substance such as metal particles for thermotherapy or radiotherapy, and a carrying body functioning as a carrier. Further, the liquid being the medium of the ejection solution is only required to be a substance suitable for administering the predetermined substance exemplified by those substances to the target region, and may be aqueous or oleaginous, which is not limited. Further, viscosity of the liquid being the medium is not particularly limited as long as the predetermined substance can be ejected by the injector 1.

In the injector 1, the igniter 22 is actuated upon by being supplied with ignition current from an external power source, to make the injector 1 inject the ejection solution. The ignition current may be supplied to the igniter 22, under various modes. For example, the power actuating the igniter 22 may be supplied from the outside through a power cable not illustrated. Alternatively, a battery for supplying the power may be provided inside the actuator 20.

Now, the actuator 20 will be described based on FIG. 2. The actuator 20 has a body 21 formed in a cylindrical shape. The body 21 includes a center portion 21a in the center thereof, a distal end portion 21b on the distal end side thereof, and a base end portion 21c on the base end side thereof. The distal end portion 21b, the center portion 21a, and the base end portion 21c of the body 21 have their internal spaces in communication with each other. The distal end portion 21b has an opening 27 on the distal end side. The igniter 22, which is an electric igniter that generates energy for ejection through combustion of an ignition charge 22a, is attached to the base end portion 21c of the body 21 via a cap 23. The igniter 22 includes an ignition pin 22b supplied with the ignition current from the outside. The attachment state of the igniter 22 to the body 21 is determined such that a combustion product generated by the operation of the igniter 22 is discharged toward the center portion 21a of the body 21. Specifically, the igniter 22 is attached to the base end portion 21c of the body 21 to have a discharge surface 22c, from which the combustion product is discharged, directed toward the center portion 21a.

Herein, combustion energy used in the igniter 22 for the ignition charge 22a is energy for the injector 1 to eject the ejection solution to the target region. Note that, examples of the ignition charge include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. An explosive other than these may be used as the ignition charge as long as appropriate ejection of the ejection solution can be performed.

The internal space of the center portion 21a of the body 21 serves as a combustion chamber 20a into which a combustion product is discharged from the igniter 22. Furthermore, a male thread portion 26 is formed in a part of the outer surface of the center portion 21a. The male thread portion 26 is configured to mate with a female thread portion 32 of the attachment 30. The effective lengths of the male thread portion 26 and the female thread portion 32 are determined to secure sufficient fastening force therebetween. The internal space of the distal end portion 21b adjacent to the center portion 21a is formed in a cylindrical shape in which a piston 40 is slidably provided and O rings 25 serving as a sealing member are also provided. In a state where the piston 40 is disposed in the internal space of the distal end portion 21b before the actuation of the actuator 20, a flange surface on the base end side of the piston 40, which serves as a surface receiving pressure of the combustion product from the igniter 22, is exposed on the combustion chamber 20a side, and the distal end of the piston 40 is accommodated in the opening portion 27.

Then, when the igniter 22 is actuated and the combustion product is discharged into the combustion chamber 20a and thus the pressure therein rises, the flange surface on the base end side of the piston 40 receives the pressure, resulting in the piston 40 sliding toward the distal end side. Thus, the actuator 20 has a mechanism with the igniter 22 serving as an actuation source and the piston 40 serving as an output unit. Since the radially expanded flange surface of the piston 40 has a larger diameter than the opening portion 27, the distance by which the piston 40 can slide is limited. Thus, the distance by which the piston 40 can protrude from the distal end surface of the distal end portion 21b of the body 21 is limited. The piston 40 may be formed of a resin, and in such a case, metal may be used together for a part where heat resistance and pressure resistance are required.

Additionally, as an alternative mechanism to adjust the pressure applied to the piston 40, the combustion chamber 20a of the actuator 20 may be further provided with a gas generating agent that is burned by the combustion product from the igniter 22 to produce gas. The agent may be disposed, for example, at a location that may be exposed to the combustion product from the igniter 22. Further, as another method, the gas generating agent may be disposed in the igniter 22 as disclosed in WO 01-031282, JP 2003-25950 A, and the like. As one example of the gas generating agent, there may be exemplified a single base smokeless explosive formed of 98 mass% of nitrocellulose, 0.8 mass% of diphenylamine, and 1.2 mass% of potassium sulfate. Further, various types of gas generating agents used in a gas generator for an air bag and a gas generator for a seat belt pretensioner may be used. A combustion completion time period of the gas generating agent can be changed by adjusting a dimension, a size, a shape, and particularly, a surface shape of the gas generating agent at the time of being disposed in the combustion chamber 20a or the like. With this, the pressure applied to the piston 40 can be adjusted to a desired pressure.

Next, the attachment 30 will be described based on FIG. 3. Note that FIG. 3 includes the diagram (a) on the left side that is a cross-sectional view of the attachment 30, and the diagram (b) on the right side that is an external view of the attachment 30. The attachment 30 is a component to be the main body of the injector 1, for attaching the actuator 20, the plunger 80, the container 70, and the like as illustrated in FIG. 1. For a body 31 of the attachment 30, nylon 6-12, polyarylate, polybutylene terephthalate, polyphenylene sulphide, a liquid crystal polymer, or the like, which are publicly known, may be used for example. Further, a filler such as glass fibers and glass filler may be contained in those resins, and from 20 to 80 mass% of glass fibers may be contained in polybutylene terephthalate, from 20 to 80 mass% of glass fibers may be contained in polyphenylene sulphide, or from 20 to 80 mass% of minerals may be contained in a liquid crystal polymer.

The internal space of the body 31 includes a first region 33, extending from the base end side to the center, where the actuator 20 is disposed as illustrated in FIG. 1. The first region 33 includes a region 33a on the base end side where the base end portion 21c of the actuator 20 is generally positioned, and a region 33b on the distal end side of the first region 33 where the center portion 21a and the distal end portion 21b of the actuator 20 are generally positioned. The region 33b has a smaller diameter than the region 33a. The female thread portion 32 is disposed on the inner wall surface at a portion of the region 33b close to the region 33a. The female thread portion 32 is formed so as to engage with the male thread portion 26 provided on the center portion 21a of the actuator 20.

Furthermore, in the internal space of the body 31, a second region 34 that is in communication with the first region 33 is formed. The second region 34 is a region in which the plunger 80 is generally disposed as illustrated in FIG. 1, and is a hollow region formed in a cylindrical shape extending along the longitudinal direction of the body 31. The second region 34 has one end in communication with the region 33b of the first region 33. The second region 34 has a diameter smaller than the diameter of the region 33b, and enables a sliding movement of the plunger 80. The plunger 80 is a member that pressurizes the ejection solution using the energy received from the piston 40, and has, on its base end side, a plunger rod 50 that receives force from the piston 40, and has, on its distal end side, a stopper portion 60 that is formed by an elastic member such as rubber and presses the ejection solution. A through hole 37 extends from a side outer surface of the attachment 30 to the second region 34, to be formed through the body 31. Through the through hole 37, the user can check the state (such as whether the injector 1 is before or after actuation, for example) of the plunger 80 in the injector 1 from the outside.

The internal space of the body 31 further includes a third region 35 in communication with the second region 34. The third region 35 is a region in which a part of the container 70 is generally disposed as illustrated in FIG. 1, and has one end in communication with the second region 34, and has the other end open to the distal end surface of the attachment 30. Specifically, the third region 35 is surrounded and defined by a wall portion 38 that is a part of the attachment 30 and an end surface 35a. The end surface 35a is a part of a surface of the attachment 30, intersecting the second region 34 in which the plunger 80 is disposed and slides. In an inner wall of the wall portion 38 of the attachment 30, a female thread portion 36 for attaching the container 70 is formed. The container 70 is attached to the attachment 30 via the female thread portion 36, using a container holder 71 described below.

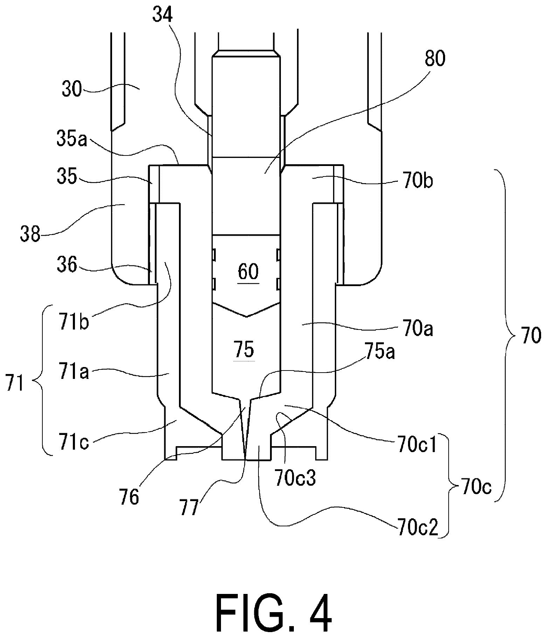

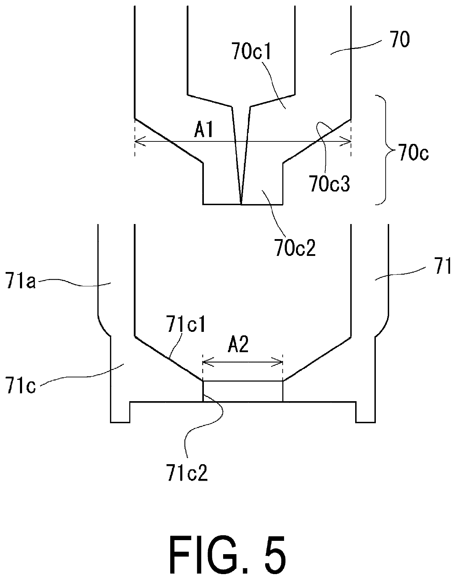

Next, attachment of the container 70 to the attachment 30 using the container holder 71 will be described based on FIG. 4 and FIG. 5. Note that FIG. 4 is a diagram illustrating an attachment state of the container 70 in the third region 35 of the attachment 30. FIG. 5 is a diagram for describing the dimensional relationship between the container 70 and the container holder 71. The container 70 is a member containing an ejection solution to be pressurized by the plunger 80, is a member that defines a flow path for ejecting the pressurized ejection solution to the target region through the ejection port, and corresponds to the housing part. In view of this, a resin material (a resin material of the same type as the attachment 30 for example) may be used for forming the container 70.

The container 70 includes an accommodating space 75 that is formed to enable the stopper portion 60 of the plunger 80 to be movable therein, and to accommodate the ejection solution, and a flow path 76 connecting the accommodating space 75 to the ejection port 77 facing the outside of the container 70. Specifically, the container 70 includes a body portion 70a having a hollow cylindrical shape defining the accommodating space 75, a base end portion 70b radially expanded to have a diameter larger than that of the body portion 70a and connected to the base end side of the body portion 70a; and a distal end portion 70c that is connected to the distal end side of the body portion 70a and defines the flow path 76. In addition, the distal end portion 70c is configured to include a protruding portion 70c2 including a part of the flow path 76 on the ejection port 77 side, and a tapered portion 70c1 that connects the protruding portion 70c2 and the body portion 70a to each other and includes a distal end side outer surface 70c3 inclined with respect to a center axis in the longitudinal direction of the attachment 30. The protruding portion 70c2 is formed to have a columnar outer circumference. The distal end side outer surface 70c3 that is the outer surface of the tapered portion 70c1 corresponding to the inner surface 75a on the distal end side of the container 70 where a communication portion at which the flow path 76 is in communication with the accommodating space 75 is located, is located more on the distal end side than the accommodating space 75 in the longitudinal direction of the attachment 30.

The container 70 itself thus formed does not have a male thread portion mating with the female thread portion 36 formed in the wall portion 38 of the attachment 30. Thus, the attachment of the container 70 to the attachment 30 is implemented using the container holder 71. The container holder 71 includes a body portion 71a defining a space incorporating the container 70, a mating portion 71b that has a male thread that mates with the female thread portion 36 formed, and is connected to the base end side of the body portion 71a, and a contact portion 71c that is connected to the distal end side of the body portion 71a, and has an inner surface 71c1 (see FIG. 5) that comes into surface contact with the distal end side outer surface 70c3 of the container 70. As illustrated in FIG. 5, the contact portion 71c is provided with an opening 71c2 through which the protruding portion 70c2 of the container 70 can pass. Here, a cross-sectional area of the body portion 70a of the container 70 is defined as A1 (A1 can be regarded as an area of a region obtained by projecting an outer profile of the body portion 70a on a plane orthogonal to the axial direction of the container 70. This will be hereinafter simply referred to as "cross-sectional area A1" in the present specification), and the area of the opening 71c2 is defined as A2.

The container 70 is accommodated with the protruding portion 70c2 passing through the opening 71c2 of the container holder 71 having such a configuration. At this time, the container 70 is in a state in which the accommodating space 75 is filled with the injection substance and the plunger 80 is loaded. In this accommodated state, the distal end side outer surface 70c3 of the container 70 and the inner surface 71c1 of the container holder 71 are in surface contact with each other on the distal end side, and the base end portion 70b of the container 70 and the mating portion 71b of the container holder 71 are in contact with each other on the base end side. While the container 70 is accommodated in the container holder 71, the male thread formed in the mating portion 71b of the container holder 71 and the female thread portion 36 of the attachment 30 are mated, until the base end side end surface of the base end portion 70b of the container 70 comes into contact with the end surface 3 5a of the third region 35 of the attachment 30. As a result, with the fastening force produced by the mating, the container 70 is attached to the attachment 30 with the distal end side outer surface 70c3 of the container 70 pressed by the container holder 71 from the distal end side toward the base end side, that is, in a direction opposite to the movement direction of the plunger 80. In this state, the plunger 80 is in a state of being located inside both the accommodating space 75 in the container 70 and in the second region 34 in the attachment 30.

The state illustrated in FIG. 1 is a state where the container 70 is thus attached to the attachment 30 by the container holder 71. In this state, when the igniter 22 of the actuator 20 is actuated, the combustion product produced therein pressurizes the piston 40, and the plunger 80 slides in the second region 34. As a result, the ejection solution accommodated in the accommodating space 75 is pressurized and ejected from the ejection port 77 through the flow path 76. The flow path 76 provided in the container 70 has a diameter smaller than the inner diameter of the accommodating space 75. With this configuration, the ejection solution that has been applied with a high pressure is ejected to the outside through the ejection port 77.

Note that the profile on the distal end side of the stopper portion 60 of the plunger 80 is shaped to generally match the profile of the inner surface 75a near a portion where the accommodating space 75 and the flow path 76 are connected to each other (the deepest part of the accommodating space 75). With this configuration, a smallest possible gap can be formed between the stopper portion 60 and the inner surface 75a of the container 70 when the plunger 80 slides when ejecting the ejection solution and reaches the deepest part of the accommodating space 75, whereby the ejection solution can be prevented from wastefully remaining in the accommodating space 75. However, the shape of the stopper portion 60 is not limited to a particular shape as long as desired effects can be obtained with the injector 1 according to the present embodiment.

When the ejection solution is thus injected, the ejection solution is pressurized by the plunger 80 within an extremely short period of time, meaning that the container 70 receives a relatively large load. However, according to the attachment mode of the container 70 according to the present embodiment, the end surface of the base end portion 70b is in surface contact with and thus is supported on the end surface 35a of the attachment 30, and the distal end side outer surface 70c3 of the distal end portion 70c is in surface contact with and is pressed and supported on the inner surface 71c1 of the container holder 71. Thus, the initial deformation of the container 70 (deformation along the movement direction of the plunger 80) can be suppressed, whereby damage or the like to the container 70 can be reliably prevented.

For the sake of suppression of the initial deformation of the container 70, the tensile strength of the material forming the container holder 71 is preferably not lower than the tensile strength of the material forming the container 70. The tensile strength is a parameter indicating the breaking strength unique to a material, which is obtained through a tensile test according to a predetermined standard such as JIS.

### Variation

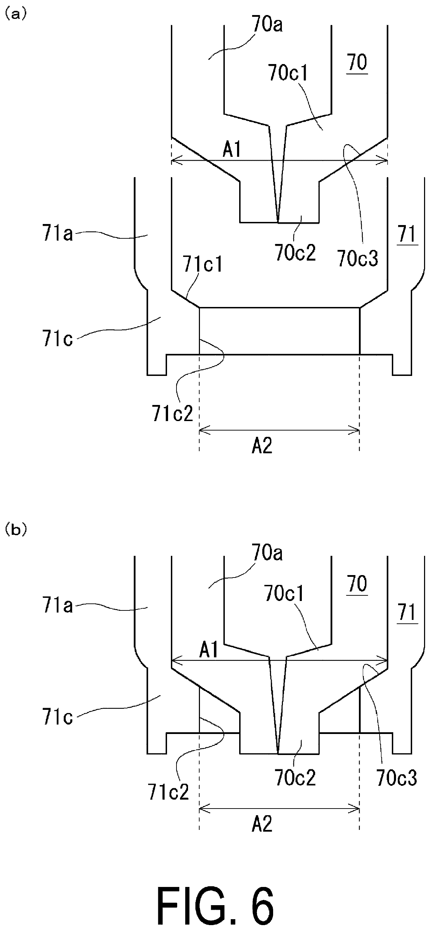

A variation of the present embodiment, related to the relationship between the container 70 and the container holder 71 in the injector 1 of the disclosure of the present application will be described based on FIG. 6. In FIG. 6, the upper section (a) illustrates a state before the container 70 is accommodated in the container holder 71, and the lower section (b) illustrates a state where the container 70 is accommodated in the container holder 71. In the variation illustrated in FIG. 6, as illustrated in the lower section (b), the contact portion 71c of the container holder 71 is in contact with a region, which is part of the distal end side outer surface 70c3 of the container 70, of a predetermined width from the connection portion between the tapered portion 70c1 and the body portion 70a, instead of the entirety of the distal end side outer surface 70c3. Also with such a relationship between the container 70 and the container holder 71, the contact portion 71c favorably presses the distal end side outer surface 70c3 when the container holder 71 mates with the attachment 30, whereby the initial deformation of the container 70 at the time of actuation of the igniter 22 can be suppressed.

### Second Embodiment

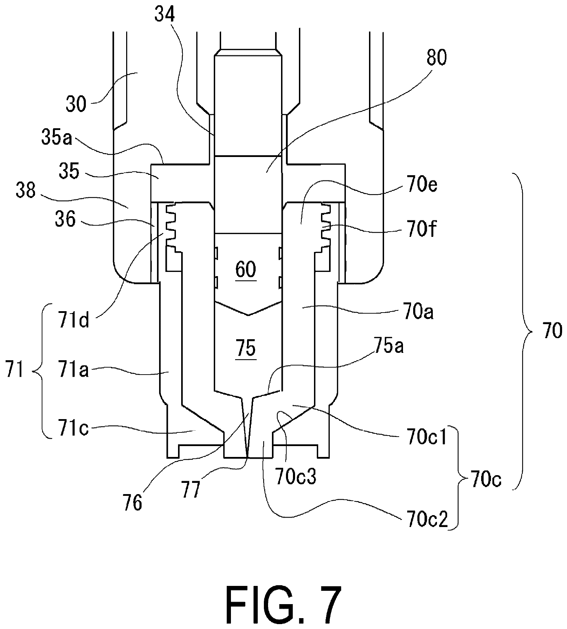

Attachment of the container 70 to the attachment 30 using the container holder 71 according to a second embodiment will be described based on FIG. 7. FIG. 7 is a diagram illustrating an attachment state of the container 70 in the third region 35 of the attachment 30 as in FIG. 4. Of the configurations illustrated in FIG. 7, those that are substantially the same as the counterparts illustrated in FIG. 4 are denoted by the same reference signs, and detailed description thereof will be omitted.

Specifically, the container 70 illustrated in FIG. 7 includes a body portion 70a having a hollow cylindrical shape defining the accommodating space 75, a base end portion 70e that is connected to the base end side of the body portion 70a, and a distal end portion 70c that is connected to the distal end side of the body portion 70a and defines the flow path 76. In the base end portion 70e, a male thread portion 70f is formed that mates with a female thread of a mating portion 71d of the container holder 71 described below. The configuration of the distal end portion 70c is substantially the same as that of the distal end portion 70c illustrated in FIG. 4. The male thread portion 70f of the container 70 does not mate with the female thread portion 36 of the attachment 30. On the other hand, the container holder 71 includes the body portion 71a defining a space incorporating the container 70, the mating portion 71d that has a male thread that mates with the female thread portion 36 of the attachment 30 and has a female thread that mates with the male thread portion 70f of the container 70 is formed, and is connected to the base end side of the body portion 71a, and the contact portion 71c that is connected to the distal end side of the body portion 71a, and has the inner surface 71c1 that comes into surface contact with the distal end side outer surface 70c3 of the container 70. The configuration of this contact portion 71c is substantially the same as the contact portion 71c illustrated in FIG. 4.

Also in the present embodiment, the container 70 is accommodated in the container holder 71, with the protruding portion 70c2 of the container 70 passing through the opening provided in the container holder 71. Here, the male thread portion 70f of the container 70 and the female thread of the mating portion 71d of the container holder 71 are mated and fastened to each other. Thus, the container 70 and the container holder 71 are integrated, in a state where the distal end side outer surface 70c3 of the container 70 is in contact with and presses against the inner surface 71c1 of the contact portion 71c of the container holder 71. Here, the plunger 80 is incorporated in the container 70, in a state where the stopper portion 60 is accommodated in the container 70. With this state maintained, the male thread of the mating portion 71d of the container holder 71 and the female thread portion 36 of the attachment 30 are mated. As result, the container 70 is attached to the attachment 30 with the distal end side outer surface 70c3 of the container 70 pressed from the distal end side toward the base end side by the container holder 71, that is, in a direction opposite to the movement direction of the plunger 80.

Also in such an attachment mode of the container 70, the distal end side outer surface 70c3 of the distal end portion 70c is in surface contact with, and thus is pressed and supported on the inner surface 71c1 of the container holder 71. Thus, the initial deformation of the container 70 at the time of actuation of the igniter 22 can be suppressed, whereby damage or the like to the container 70 can be reliably avoided. Also in the present embodiment, the contact portion 71c of the container holder 71 is formed to be in contact with part of the distal end side outer surface 70c3 of the container 70 as illustrated in FIG. 6.

### Variation

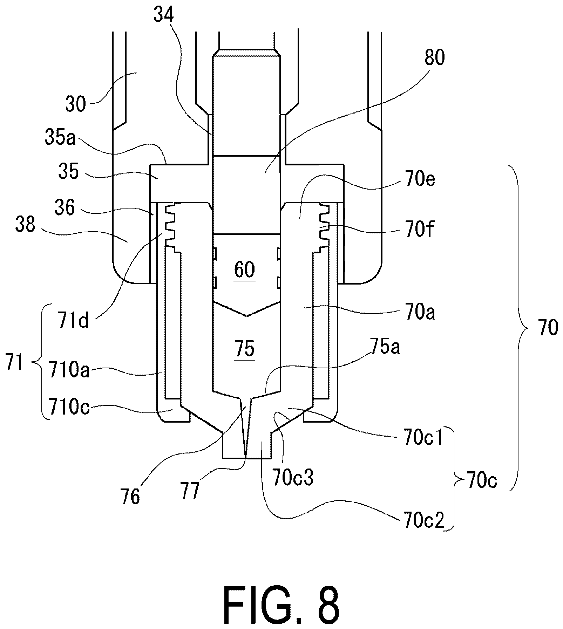

A variation of the present embodiment related to the shape of the container holder 71 will be described based on FIG. 8. FIG. 8 is a diagram illustrating an attachment state of the container 70 in the third region 35 of the attachment 30 as in FIG. 7. Of the configurations illustrated in FIG. 8, those that are substantially the same as the counterparts illustrated in FIG. 7 are denoted by the same reference signs, and the detailed description thereof will be omitted. The container holder 71 illustrated in FIG. 8 is different from the container holder 71 illustrated in FIG. 7 in terms of the shape of a body portion 710a and a contact portion 710c.

In the present variation, the body portion 710a of the container holder 71 defines a space incorporating the container 70, with no contact between the inner surface of the body portion 710a and the outer surface of the body portion 70a of the container 70, and with a predetermined gap formed therebetween. The contact portion 710c is formed to be in contact with a predetermined region that is part of the distal end side outer surface 70c3 of the container 70, that is, a region of a predetermined width not covering the entirety of the distal end side outer surface 70c3 from the connection portion between the body portion 70a and the distal end portion 70c.

Also in such an attachment mode of the container 70 using the container holder 71, the distal end side outer surface 70c3 of the distal end portion 70c is in surface contact with, and thus is pressed and supported on the inner surface of the container holder 710c. Thus, the initial deformation of the container 70 at the time of actuation of the igniter 22 can be suppressed, whereby damage or the like to the container 70 can be reliably avoided.

### Examples

Now, a description will be given on a specific dimensional relationship between the container 70 and the container holder 71, for favorably suppressing the initial deformation of the container 70. The cross-sectional area A1 of the body portion 70a is an important parameter regarding the container 70. The area A2 of the opening 71c2 is an important parameter regarding the container holder 71. The inventor of the invention according to the present application has found that the initial deformation of the container 70 is favorably suppressed, when a ratio of the area A2 to the cross-sectional area A1 (hereinafter, referred to as "area ratio A2/A1") is within a predetermined range.

Specifically, the area ratio A2/A1 is preferably a numerical value that falls within a range of from 0.10 to 0.74. Preferably, the area ratio A2/A1 is a numerical value that falls within a range of from 0.15 to 0.67. On the other hand, it has been further found that, when only the side surface of the body portion 71a of the container 70 is reinforced by the container holder 71 without pressing and reinforcing the distal end side outer surface 70c3 of the container 70 as in a known technique, that is, when the area ratio A2/A1 is approximately 1 with the container holder 71 not being provided with the inner surface 71c1, it is difficult to sufficiently suppress damage or the like to the container 70.

Each aspect disclosed in the specification of the present application can be combined with any other feature disclosed herein.

### Reference Signs List

1 Injector (needleless injector)
20 Actuator
22 Igniter
30 Attachment
33 First region
34 Second region
35 Third region
35a End surface
40 Piston
70 Container
70a Body portion
70b Base end portion
70c Distal end portion
70c1 Tapered portion
70c2 Protruding portion
70c3 Distal end side outer surface
70e Base end portion
70f Male thread portion
71 Container holder
71a Body portion
71b Mating portion
71c Contact portion
71c2 Opening
71d Mating portion
710a Body portion
710c Contact portion
75 Accommodating space
76 Flow path
77 Ejection port
80 Plunger

## Claims

1. A needleless injector configured to eject an injection substance to a target region without using an injection needle, the needleless injector comprising:
an injector main body;
a housing part including an accommodating space in which the injection substance is accommodated, the housing part defining a flow path to enable the injection substance to be ejected to the target region through an ejection port;
an attachment part configured to attach the housing part to the injector main body;
a driving part that is provided in the injector main body and imparts ejection energy for ejecting the injection substance; and
a pressurizing unit that is disposed to be movable in a direction toward the ejection port in a predetermined region that is provided to be located inside both the injector main body and the housing part, and pressurizes the injection substance accommodated in the accommodating space, upon being imparted with the ejection energy, wherein
the attachment part attaches the housing part to the injector main body, with a distal end side outer surface of the housing part corresponding to an inner surface of the housing part on a distal end side, where a communication portion where the flow path is in communication with the accommodating space is located, pressed in a direction opposite to a movement direction of the pressurizing unit toward the ejection port.

2. The needleless injector according to claim 1, wherein
the housing part includes a base end portion radially expanded in a radial direction and including a base end surface, a distal end portion including the distal end side outer surface, and a body portion that connects the base end portion and the distal end portion to each other, and
the attachment part is fastened to the injector main body with the base end portion sandwiched between the attachment part and the injector main body, to attach the housing part to the injector main body with the base end surface of the housing part on a side opposite to a distal end surface of the housing part where the ejection port is provided, brought into contact with a predetermined end surface that is part of a surface of the injector main body intersecting the predetermined region, and with the distal end side outer surface of the housing part pressed.

3. The needleless injector according to claim 1, wherein
the housing part includes a base end portion where the attachment part is fastened to the housing part, the base end portion including a base end surface, a distal end portion including the distal end side outer surface, and a body portion that connects the base end portion and the distal end portion to each other,
the attachment part is fastened to the housing part to make the attachment part be in contact with and press the distal end side outer surface of the distal end portion, and
the housing part is attached to the injector main body with the attachment part to which the housing part is fastened being fastened to the injector main body.

4. The needleless injector according to claim 2 or 3, wherein
the distal end portion includes a protruding portion that includes part of the flow path on a side of the ejection port; and a tapered portion that connects the protruding portion and the body portion to each other and includes the distal end side outer surface inclined with respect to a center axis along a longitudinal direction of the injector main body, and
the attachment part has an opening formed, through which the protruding portion passes, to open the ejection port, and includes a contact portion that comes into contact with a region having a predetermined width from a connection portion where the tapered portion and the body portion are connected to each other in the distal end side outer surface.

5. The needleless injector according to claim 4, wherein a ratio of an area of the opening to a cross-sectional area of the body portion is from 0.10 to 0.74.

6. The needleless injector according to any one of claims 1 to 5, wherein tensile strength of the attachment part is not lower than tensile strength of the housing part.
